# EUROPEAN PATENT SPECIFICATION

(11) **EP 2 663 238 B1**
(45) Date of publication and mention of the grant of the patent: **09.12.2020**
(21) Application number: 12700143.6
(22) Date of filing: 04.01.2012
(51) Int. Cl.: A61B 17/00, B44F 1/10

(54) **SURGICAL INSTRUMENT WITH HIGH CONTRAST MARKING AND METHOD OF MARKING A SURGICAL INSTRUMENT**
CHIRURGISCHES INSTRUMENT MIT MARKIERUNG MIT HOHEM KONTRAST UND VERFAHREN ZUR MARKIERUNG EINES CHIRURGISCHEN INSTRUMENTS
INSTRUMENT CHIRURGICAL À MARQUAGE À CONTRASTE ÉLEVÉ ET PROCÉDÉ DE MARQUAGE D'UN INSTRUMENT CHIRURGICAL

(30) Priority: 11.01.2011 GB 201100405
(43) Date of publication of application: 20.11.2013
(73) Proprietor: DePuy Ireland Unlimited Company, County Cork (IE)
(72) Inventor: COULTRUP, Oliver, Yorkshire LS11 8DT (GB); YOUNG, Duncan, Yorkshire LS11 8DT (GB)
(74) Representative: Pawlyn, Anthony Neil
(86) International application number: PCT/GB2012/050004
(87) International publication number: WO 2012/095642

(56) References cited:
- EP-A1- 0 358 495
- EP-A1- 0 578 027
- ES-A1- 2 042 423
- GB-A- 416 350
- JP-A- 2007 168 341
- US-A1- 2003 009 177
- US-A1- 2007 291 320

## Description

The present invention relates to a surgical instrument which includes a debossed symbol formed on a surface to give improved contrast with the surface. The present invention also relates to a method of marking a surgical instrument with a debossed symbol.

It is usually desired to mark a surgical instrument in some way. Typically, the marking will involve symbols including alphanumeric characters or other typographical symbols, manufacturing designations, logos, and other useful information.

In order to obtain regulatory approval of a surgical instrument, it is necessary to show that the nature of any marking is biocompatible. This is particularly important when surgical instruments are marked using inks or dyes; the biocompatibility of the inks or dyes must be shown. It also applies to alternative methods such as laser etching. Laser etching may alter the chemical properties of the instrument in the vicinity of the mark and these have to be shown as biocompatible.

It is known to mark symbols on surgical instruments using debossing or embossing. A debossed symbol is a symbol which is stamped or otherwise formed in the surface of the surgical instrument, so that the debossed symbol defined a recess below the surface of the surgical instrument. An embossed symbol is a symbol formed on or otherwise raised above the surface of the surgical instrument.

A debossed or embossed symbol is typically made of the same material as the part of a surgical instrument it is formed in. Therefore any problems with biocompatibility are avoided. However, conventional debossed or embossed symbols can be difficult for a surgeon to read in the operating theatre. The debossed or embossed symbol is formed from the same material as the surface surrounding it and therefore is substantially the same colour and shade as the surrounding surface. This can make it difficult to identify a symbol during a surgical procedure. It is important that symbols imparting information important for the surgical procedure, such as the size of an instrument, can be read clearly by a user. Therefore, the present invention aims to improve the clarity of a symbol on a surgical instrument.

US-A-2007/0291320 (Chen) discusses a wedged surface structure. Some of the ridges may be cut at one or both lengthwise upper edges to produce bevel surfaces at different positions. The bevel surfaces produce at least one engraved pattern and multiple overlapped engraved patterns can be provided on a ridged surface. Which engraved pattern is seen depends on a viewing angle of a user.

In EP-A-0358495 (Daly) decorative elements are formed with a plurality of elongate surface projections or depressions. Portions of the outer faces of the projections or depressions are formed in different colours so that they appear to have a different colour depending on the viewing angle of the user.

Neither Chen nor Daly suggest how to improve the contrast of a character from all viewing angles.

EP0578027 discloses an embossed marking standing above the surrounding flat surface, which can be used as an information marker for tubular objects

### SUMMARY

Claims 1 and 8 define the invention and the dependent claims disclose the preferred embodiments. According to a first aspect of the present invention, there is provided a surgical instrument according to claim 1.

The depth of the trough provides a sufficient size of facet that the shadow and reflection effects provide a useful enhancement to the contrast of the symbol.

The ridges and grooves create an improved contrast for the symbol relative to the surrounding surface by a combination of different specular reflection of incident light compared to the surrounding surface and shadows formed by the ridges and grooves.

Although marking systems using ridges and grooves have been proposed in other fields than surgical instruments, these marking systems do not improve contrast of a symbol relative to a smooth surrounding surface. Instead they seek to alter the appearance of the surface depending on the viewing angle. This is not the same as the present invention, where contrast is improved regardless of viewing angle and the symbol retains substantially the same appearance regardless of viewing angle.

The groove may extend completely through the surgical instrument. In that case, where adjacent angled surfaces define triangles in cross section, there will be a gap between each pair of angled surfaces.

The grooves are at a greater depth than the ridges. The angled surfaces may be substantially flat or curved. The reference to a "smooth surface" means free of ridges and grooves; some element of roughness may remain. The smooth surface may be substantially flat or non-flat, for the smooth surface may be substantially planar or curved or rounded. The symbol may be any alphanumeric character, typographical character, logo or other graphical item.

The angled surfaces preferably extend over the entire area of the symbol, although this is not essential to the invention. The angled surfaces are preferably angled relative to the smooth surface.

The ridges and grooves may be parallel to the smooth surface, if it was extended over the area of the symbol. For example, when the smooth surface is substantially flat or planar, the angled surfaces may be provided by substantially planar facets. Alternatively, when the smooth surface is curved, the angled surfaces may also be curved.

Unlike the prior art, such as Chen and Daly discussed above, which apply ridges and grooves to an element to present different patterns or colours depending on viewing angle, the present invention enhances contrast compared with the surrounding surface regardless of viewing angle.

The angled nature of the angled surfaces defining the ridges and grooves means that incident light is reflected differently from the facets than the surrounding surface. In addition, the peaks of the ridges form shadows which further act to increase contrast. Depending on the precise size of the facets the increased contrast may appear as a single, generally uniform shading difference because the eye will blend the different reflection and shadow areas to a single shading. Alternatively, if the facets are relatively large, the contrast may be enhanced by the appearance of different bands of alternate shading within the debossed character arising from the reflection and shadow effects.

Preferably, adjacent angled surfaces meet at an edge which is sharp. A sharp edge maximises the improved contrast effect. Typically, a sharp edge will have a radius of curvature less than 0.2mm, more preferably less than or equal to about 0.12mm. It is generally preferred that the radius of curvature is as small as possible, the actual value that can be achieved is likely to depend on the manufacturing route used.

In a cross-section through the symbol in a plane perpendicular to the ridges and grooves, alternating angled surfaces extend from a trough of a groove to a peak of a ridge and from a peak of a ridge to a trough of a groove respectively. This creates a generally triangular wave pattern. Such a pattern of angled surfaces can be manufactured simply.

Preferably, in a cross-section through the symbol in a plane perpendicular to the direction of the peak of the ridges and the trough of the grooves, each pair of adjacent angled surfaces defines two equal sides of an isosceles triangle. Preferably, the angled surfaces are angled at about 90° to each other and about 45° relative to the plane of the debossed symbol. This equalises the contrast effect between reflection and shadow areas within the debossed symbol. Adjacent pairs of angled surfaces may define two sides of a triangle other than an isosceles triangle and other angles can be used.

Preferably, the plurality of angled surfaces have a different surface roughness than the smooth surface. The plurality of angled surfaces may have a smaller surface roughness than the smooth surface. The plurality of the angled surfaces may have a greater surface roughness than the smooth surface. For example, the angled surfaces or the smooth surface may be polished to reduce surface roughness. Polishing can be achieved by polishing after formation or during formation. For example, moulds can be used in which the angled surface forming elements of the mould or the smooth surface forming elements of the mould are polished. The surface roughness of the polished element may have a Rₐ of less than about 1.8 µm, more preferably between about 1.12 µm and about 1.65 µm. This compares with a typical surface roughness giving an Rₐ of around 6.3 µm for an unpolished element.

The symbol may be a typographic symbol and the plurality of ridges may then be substantially parallel to a horizontal element of the typographic symbol. The reference to a horizontal element refers to the horizontal strokes of the typographical symbol, for example a "-" is a horizontal element.

Preferably, the spacing between peaks of adjacent ridges is between 0.5mm and 2mm, more preferably about 1mm.

Advantageously, at least three ridges are defined by the plurality of angled surfaces within the area of the symbol. Depending on the size of the symbol, more ridges may be defined. However, it is preferred to have at least three ridges to ensure that the contrast enhancement effect is useful.

Preferably, the symbol does not comprise any printed markings. More preferably, the entire surgical instrument does not include any printed markings to avoid any difficulty with biocompatibility of marking materials.

The peak of the ridges may be at the same level as the smooth surface, or at a lower level than the smooth surface. Less preferably, the peak of the ridges may extend above the smooth surface.

Another aspect of the invention provides a method of making a surgical instrument according to claim 8.

If the symbol is formed by moulding, then the steps of forming a symbol and forming a plurality of angled surfaces can take place substantially simultaneously in a single moulding step. For example, the mould may include angled surfaces in the symbol.

Preferably, the mould is selectively polished or roughened to produces a different surface roughness in the plurality of facets compared to the smooth surface. Either the angled surfaces or the surrounding surface in the mould may be polished, to produce a different surface roughness between the surrounding surface and the angled surfaces. Alternatively, either the surrounding surface or angled surfaces may be roughened in the mould to produce a different surface roughness between the surrounding surface and the angled surfaces. One of the surrounding surface and the angled surfaces may be roughened in the mould and the other of the surrounding surface and the angled surfaces forming the symbol may be polished in the mould to produce a different surface roughness between the surrounding surface and the angled surfaces.

If the symbol is formed by machining, then the steps of forming a symbol and forming a plurality of angled surfaces may take place substantially simultaneously in a single machining step. In other words, the machining of the symbol may at the same time machine the angled surfaces into the symbol. The method may further comprise selectively polishing or roughening the plurality of angled surfaces or the smooth surface after formation of the symbol to produce a different surface roughness between the smooth surface compared to the plurality of angled surfaces.

Embodiments of the invention will now be described by way of example only with reference to the accompanying drawings, in which:
Figure 1 depicts a top view of a tibial sizing guide according to a first embodiment of the present invention;
Figure 2 depicts a bottom view of the tibial sizing guide of Figure 1;
Figure 3 depicts a cross-section through a vertical stroke of a "four" symbol marked on the tibial sizing guide of Figure 1;
Figures 4A-4G are a series of photographs of example tibial sizing guides with debossed symbols containing ridges and grooves according to the present invention and prior art plain debossed symbols at a variety of viewing angles;
Figures 5-9 give examples of numeric debossed characters with ridges and grooves formed according to alternative embodiments of the present invention; and
Figure 10 depicts a perspective view of a tibial keel punch according to another embodiment of the invention.

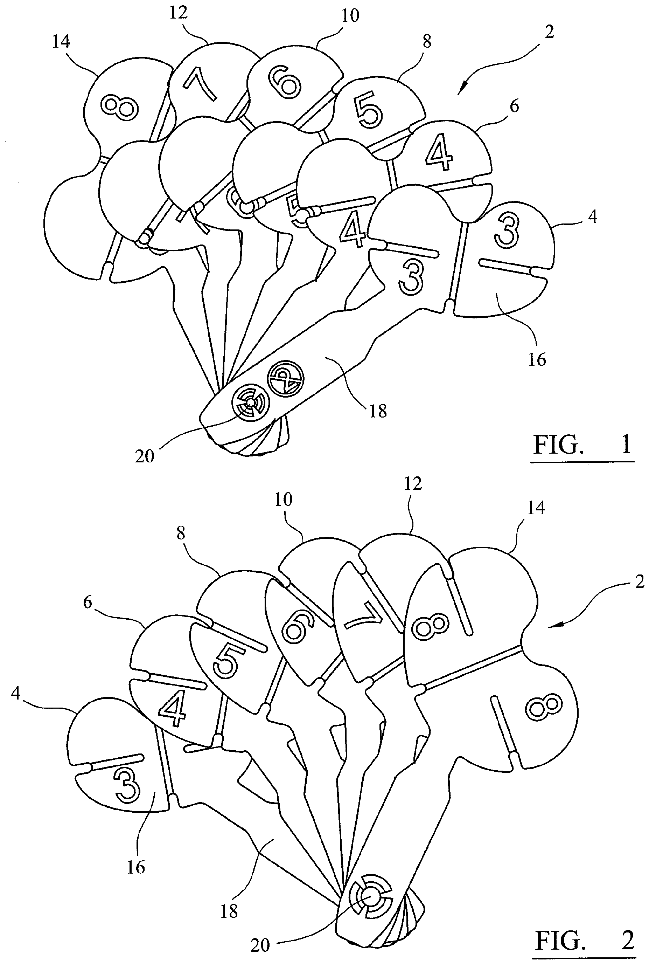

Figure 1 depicts a top view of a tibial sizing guide 2 according to a first embodiment of the present invention. Figure 2 depicts the tibial sizing guide of Figure 1 from the bottom. The tibial sizing guide 2 comprises six separate tibial sizing guides 4, 6, 8, 10, 12, 14. Each tibial sizing guide contains a planar sizing element 16 extending from a shaft 18. The tibial sizing guides are joined along an axis 20 perpendicular to the plane of the sizing element 16. In use, the planar sizing element 16 is used to measure the size of a tibia during knee surgery. Each tibial sizing guide, 4, 6, 8, 10, 12, 14 is marked with a debossed symbol indicating the size it represents. In this case the symbol is a numeric character.

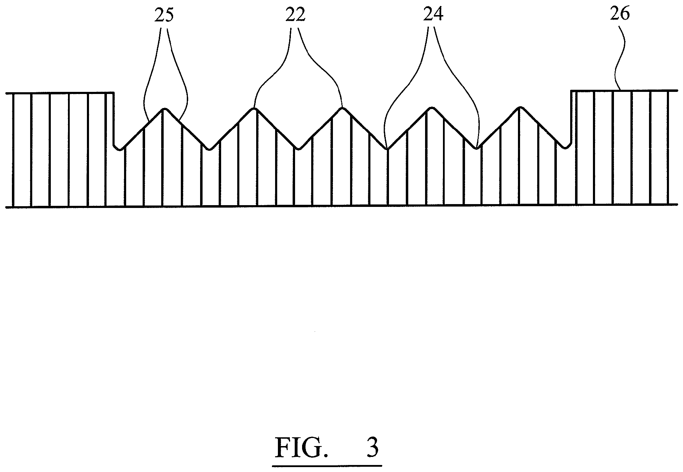

The debossed symbols are provided with a plurality of ridges and grooves, described in more detail below with reference to Figure 3. For clarity in Figures 1 and 2, debossed symbols on the tibial sizing guide which include the plurality of ridges and grooves are marked in black.

Figure 3 is a diagrammatic representation (not to scale) of a partial cross section through a vertical stroke of a "four" character in the embodiment of Figures 1 and 2. Figure 3 depicts how the debossed symbol contains a plurality of ridges 22 and grooves 24 extending over the area of the debossed symbol. In this embodiment the depth of the trough of the grooves 24 is about 0.6mm from the smooth surface and the height of the peak of the ridges 22 above the trough of the grooves 24 is about 0.1mm. The peaks of the ridges 22 in this embodiment are therefore below the level of the surrounding surface 26 in which the debossed symbol is formed. In other embodiments different dimensions may be used as appropriate.

A single angled surface extends from the peak of each ridge 22 to the trough of each groove 24. In this embodiment the angled surface is a substantially planar facet 25. The angle between adjacent facets 25 is about 90°. As can be seen in Figure 3, in this embodiment the debossed symbol, and the trough of the grooves do not extend through the entire depth of the part of the surgical instrument in which they are formed. This means that the debossed characters are invisible from the underside.

The facets 25 forming the ridges 22 and the grooves 24 are preferably polished.

In use, the angling of the facets 25 relative to the surrounding surface 26 creates a combination of different specular reflection and shadows caused by incident light. In an operating theatre, incident light is generally from above, designed to illuminate the operating area evenly. This gives surgical lighting a directional quality that improves the enhanced contrast of the present invention under surgical lighting conditions. In use, the facets 25 create a pattern of reflection and shadow which alters the perceived shade of the debossed symbol relative to the surrounding surface.

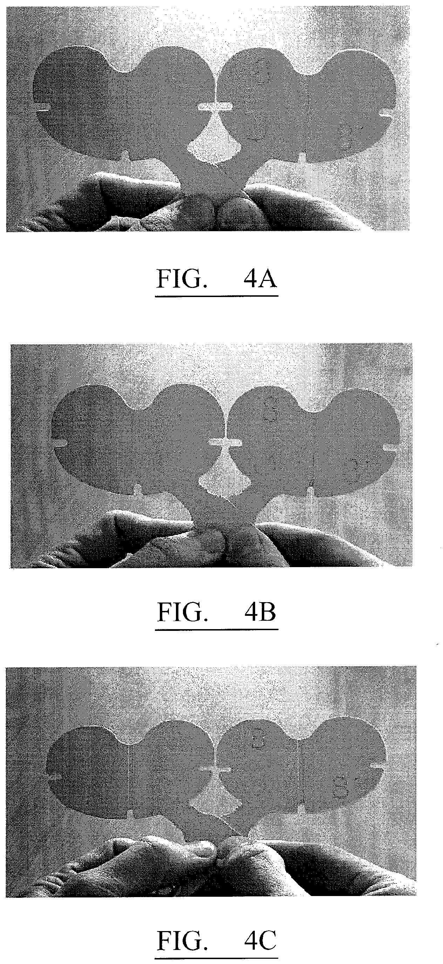

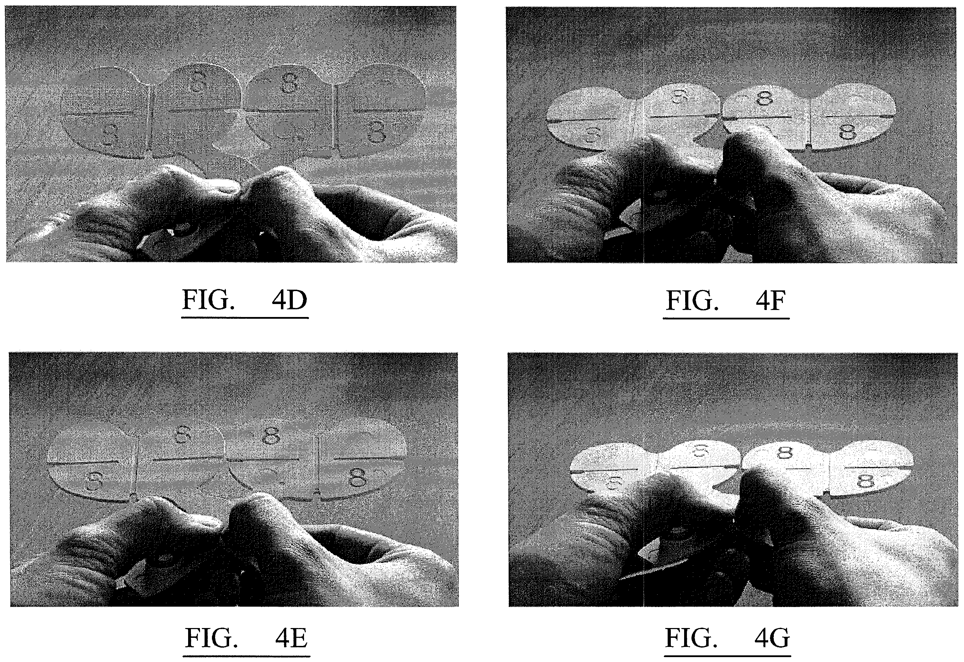

Figures 4A-4G are a series of photos showing the improved contrast of a debossed symbol with ridges and grooves according to the present invention compared with a prior art plain debossed symbols. On the instrument on the right hand side of the photos a debossed symbol with ridges and grooves of the invention is depicted. On the left hand side, a conventional surgical instrument without the ridges and grooves in its debossed symbols is depicted. It can be seen how the debossed symbol of the invention on the right hand side instrument, including ridges and grooves, has improved contrast versus the prior art symbol at virtually all angles relative to an onlooker.

Although this embodiment has been described in terms of a tibial sizing guide, it will be appreciated that the improved contrast of the present invention is advantageous when applied to any surgical instrument, not just tibial sizing guides.

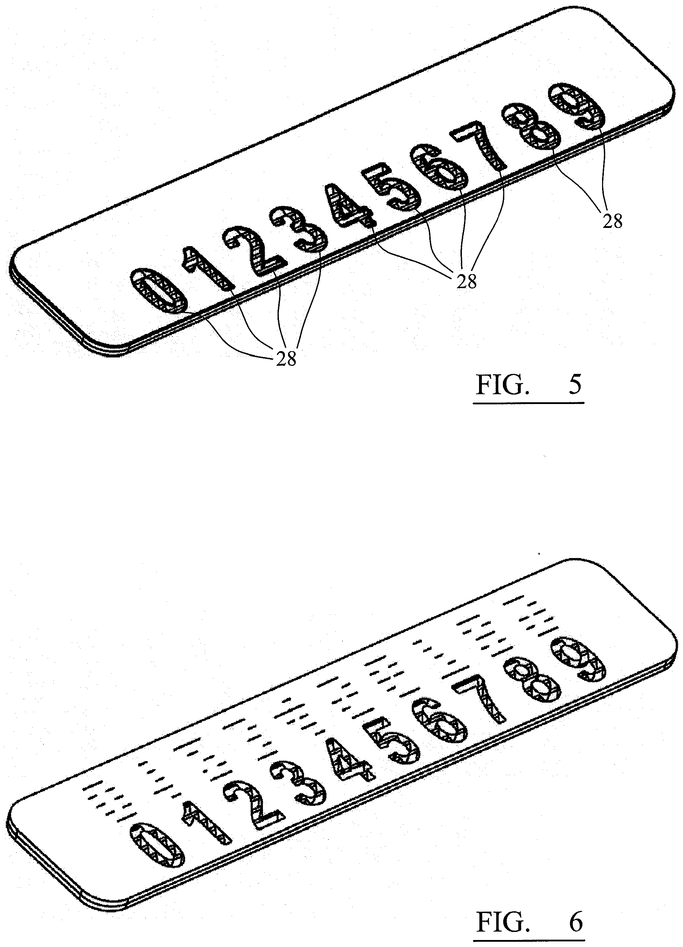

Figures 5-9 depict examples of debossed numerical characters according to further embodiments of the invention. To understand the effect of extending the debossed character through the entire depth of the component to be marked, Figures 5-9 depict numerical symbols debossed on a side visible to an onlooker at the bottom. At the top, debossed symbols formed on the opposite to the onlooker are depicted.

In Figure 5 the grooves of the facets formed within the debossed numerical characters 28 do not extend through the entire depth of the material. Therefore, no evidence of the corresponding debossed characters marked on the underside of the example surface in Figure 5 can be seen.

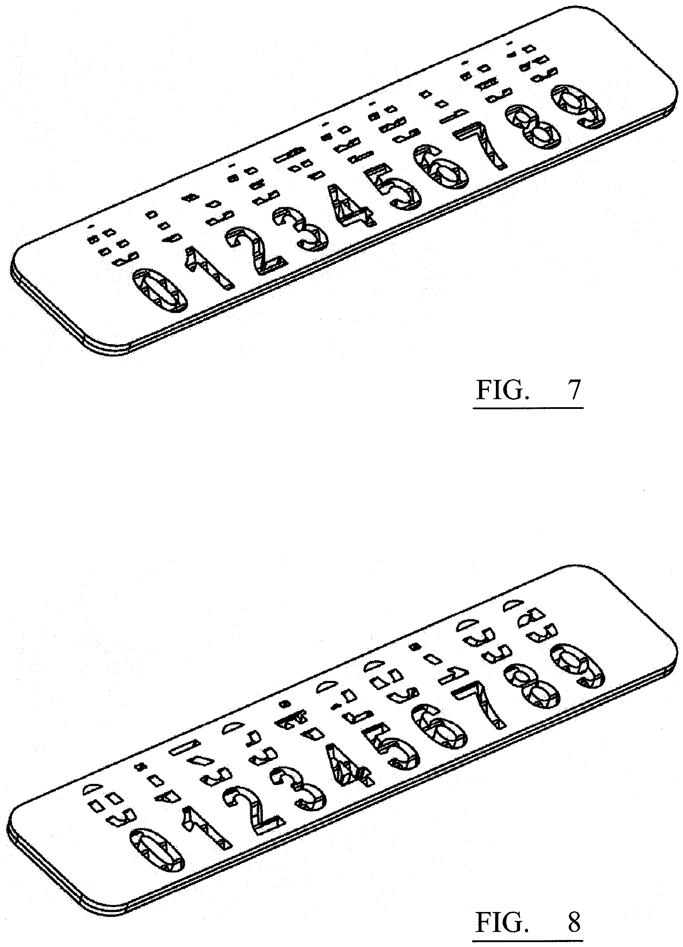

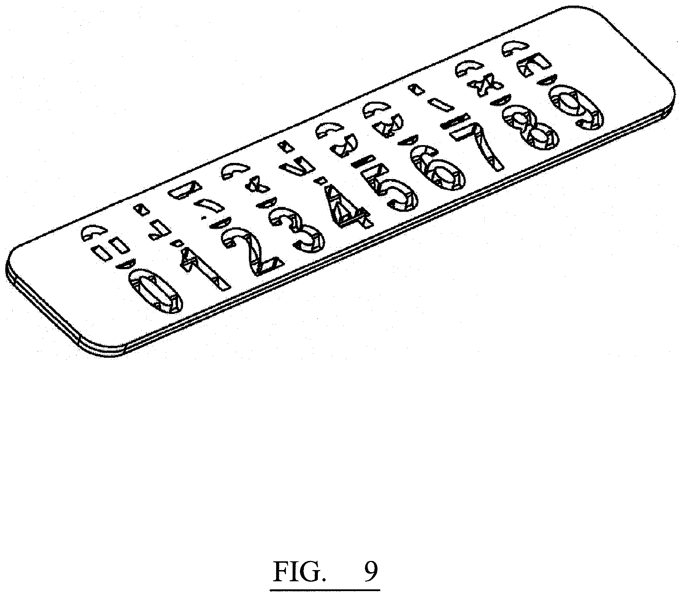

In the embodiments of Figures 6-9 the grooves extend through the entire depth of the component in which the debossed numerical characters are marked. The spacing between alternating pairs of facets either side of a groove which extends through the entire depth of the substrate is increased in the embodiment of Figure 7 compared with Figure 6, further increased in the embodiment of Figure 8 compared with Figure 7 and still further increased in the embodiment of Figure 9 compared with Figure 9.

Figures 6-9 show how, even when the troughs of the groove extend through the entire depth of the component and occupy a significant area of the numerical character, there is a low risk of confusing a character marked on the opposite surface with a character marked on the surface viewed by an onlooker.

In the embodiments of Figures 5-9, the details of the surgical instrument to which the debossed symbols are applied has not been shown. For example, the numerical characters depicted in these embodiments could be applied to the numerical markings on the tibial sizing guide of Figures 1 and 2, as well as any other surgical instrument. It will also be appreciated that the concepts of Figures 5-9 can be applied to any symbol and is not limited to numerical characters.

The present invention is preferably manufactured from injection moulded plastics material. In that case, the debossed characters and facets can be defined in a single injection moulding step. Other moulding processes may also be used. Alternatively, the debossed characters may be formed by machining. This can be useful when the surgical instrument is manufactured of other materials than plastics materials, for example, metal alloys. The invention can also be applied to ceramic materials. Other production processes may also be used, including casting, metal injection moulding or stamping.

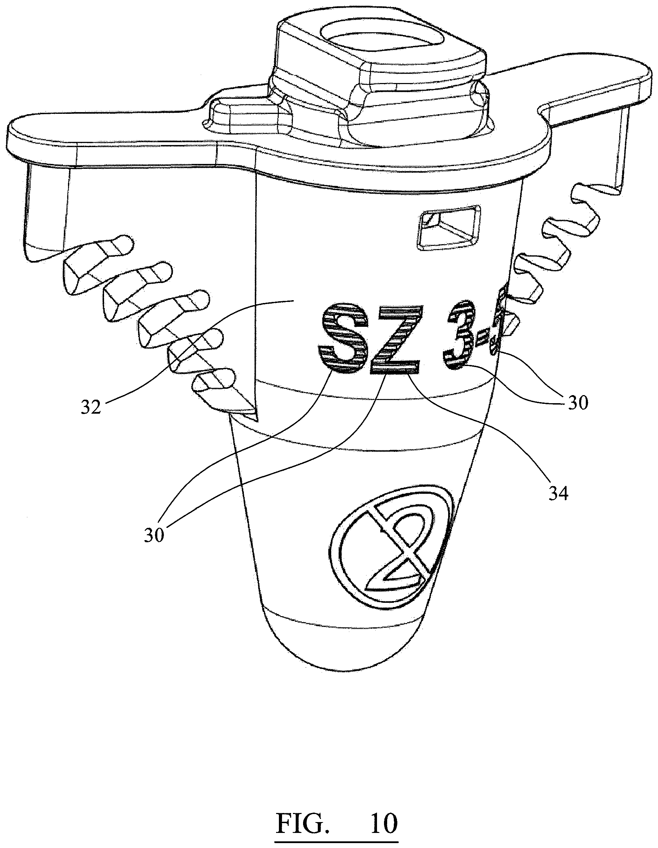

A further embodiment of the invention is depicted in Figure 10. This depicts a tibial keel punch having debossed symbols 30 provided on a curved surface 32. The construction of this embodiment is the same as described above, apart from the debossed symbols 30 are provided on a curved surface rather than a flat surface. The ridges and grooves defined by the angled surfaces 34 of the debossed symbols 30 are also curved, generally following the shape of the curved surface 32 if it were extended over the debossed symbol. The ridges and grooves defined by the angled surfaces remain parallel to each other.

To improve the contrast effect of the present invention it is preferred to use a lighter rather than a darker material so that the shadows and highlights produced by the facets create a greater difference in contrast. However, the invention still has an effect with all colours and shades.

While the above described embodiments have discussed debossed symbols, it will be appreciated that the construction and techniques discussed can equally be applied to embossed symbols, and will improve the contrast of an embossed symbol relative to a surrounding surface.

## Claims

1. A surgical instrument (2) comprising a debossed symbol formed in or on a smooth surface (26) surrounding the symbol, wherein the symbol comprises a plurality of angled surfaces over at least part of the area of the symbol, wherein the angled surfaces (25) define a plurality of parallel ridges (22) and grooves (24) in the symbol, and wherein, in a cross section through the symbol in a plane perpendicular to the ridges and grooves, alternating angled surfaces extend from a trough of a groove to a peak of a ridge and from a peak of a ridge to a trough of a groove, respectively, and wherein the depth of a trough of a groove is at least 0.5mm.

2. A surgical instrument (2) according to claim 1, wherein the spacing between peaks of adjacent ridges (22) is between 0.5mm and 2mm.

3. A surgical instrument (2) according to claim 2, wherein the spacing between peaks of adjacent ridges (22) is 1mm.

4. A surgical instrument (2) according to any one of the preceding claims, wherein the plurality of angled surfaces (25) have a different surface roughness than the smooth surface (26).

5. A surgical instrument (2) according to claim 4, wherein the surface roughness has an Rₐ of less than 1.8µm.

6. A surgical instrument (2) according to any one of the preceding claims, wherein at least three ridges (22) are defined by the plurality of angled surfaces (25).

7. A surgical instrument according to any one of the preceding claims, wherein the symbol does not comprise any printed markings.

8. A method of marking a surgical instrument (2) with a debossed symbol formed in or on a smooth surface (26), the method comprising:
forming a debossed symbol in or on a smooth surface of the surgical instrument; and
forming a plurality of angled surfaces (25) over at least part of the area of the symbol, wherein the angled surfaces define a plurality of parallel ridges (22) and grooves (24) in the symbol wherein the angled surfaces define a plurality of parallel ridges and grooves in the symbol, the angled surfaces defining a plurality of parallel ridges and grooves in the symbol, and wherein, in a cross section through the symbol in a plane perpendicular to the ridges and grooves, alternating angled surfaces extend from a trough of a groove to a peak of a ridge and from a peak of a ridge to a trough of a groove, respectively, and wherein the depth of a trough of a groove is at least 0.5mm.

9. A method according to claim 8, wherein the symbol is formed by moulding and the steps of forming a debossed symbol and forming a plurality of angled surfaces (25) take place substantially simultaneously in a single moulding step.

10. A method according to claim 9, wherein the mould is selectively polished or roughened to produce a different surface roughness of the plurality of angled surfaces (25) compared to the smooth surface (26).

11. A method according to claim 8, wherein the symbol is formed by machining and the steps of forming a debossed symbol and forming a plurality of angled surfaces (25) take place substantially simultaneously in a single machining step.

12. A method according to claim 11, further comprising selectively polishing or roughening the plurality of angled surfaces (25) or the smooth surface (26) after formation of the symbol to produce a different surface roughness of the smooth surface compared to the plurality of angled surfaces.

## Patentansprüche

1. Chirurgisches Instrument (2) umfassend ein tiefgeprägtes Symbol, das in oder auf einer glatten Oberfläche (26) ausgebildet ist, die das Symbol umgibt, wobei das Symbol eine Mehrzahl abgewinkelter Oberflächen über zumindest einen Teil des Bereichs des Symbols aufweist, wobei die abgewinkelten Oberflächen (25) eine Mehrzahl paralleler Rippen (22) und Rillen (24) in dem Symbol definieren, und wobei in einem Querschnitt durch das Symbol in einer Ebene senkrecht zu den Rippen und Rillen sich abwechselnd abgewinkelte Oberflächen von einer Mulde einer Rille zu einer Spitze einer Rippe bzw. von einer Spitze einer Rippe zu einer Mulde einer Rille erstrecken, und wobei die Tiefe einer Mulde einer Rille zumindest 0,5 mm beträgt.

2. Chirurgisches Instrument (2) nach Anspruch 1, wobei der Abstand zwischen Spitzen benachbarter Rippen (22) zwischen 0,5 und 2 mm beträgt.

3. Chirurgisches Instrument (2) nach Anspruch 2, wobei der Abstand zwischen Spitzen benachbarter Rippen (22) 1 mm beträgt.

4. Chirurgisches Instrument (2) nach einem der vorhergehenden Ansprüche, wobei die Mehrzahl abgewinkelter Oberflächen (25) eine andere Oberflächenrauigkeit aufweisen als die glatte Oberfläche (26).

5. Chirurgisches Instrument (2) nach Anspruch 4, wobei die Oberflächenrauigkeit einen Rₐ von weniger als 1,8 µm aufweist.

6. Chirurgisches Instrument (2) nach einem der vorhergehenden Ansprüche, wobei zumindest drei Rippen (22) durch die Mehrzahl abgewinkelter Oberflächen (25) definiert sind.

7. Chirurgisches Instrument nach einem der vorhergehenden Ansprüche, wobei das Symbol keine gedruckten Markierungen umfasst.

8. Verfahren zum Markieren eines chirurgischen Instruments (2) mit einem tiefgeprägten Symbol, das in oder auf einer glatten Oberfläche (26) ausgebildet ist, wobei das Verfahren umfasst:
Ausbilden eines tiefgeprägten Symbols in oder auf einer glatten Oberfläche des chirurgischen Instruments; und
Ausbilden einer Mehrzahl abgewinkelter Oberflächen (25) über zumindest einen Teil des Bereichs des Symbols, wobei die abgewinkelten Oberflächen eine Mehrzahl paralleler Rippen (22) und Rillen (24) in dem Symbol definieren, wobei die abgewinkelten Oberflächen eine Mehrzahl paralleler Rippen und Rillen in dem Symbol definieren, wobei die abgewinkelten Oberflächen eine Mehrzahl paralleler Rippen und Rillen in dem Symbol definieren, und wobei in einem Querschnitt durch das Symbol in einer Ebene senkrecht zu den Rippen und Rillen sich abwechselnd abgewinkelte Oberflächen von einer Mulde einer Rille zu einer Spitze einer Rippe bzw. von einer Spitze einer Rippe zu einer Mulde einer Rille erstrecken, und wobei die Tiefe einer Mulde einer Rille zumindest 0,5 mm beträgt.

9. Verfahren nach Anspruch 8, wobei das Symbol durch Formen gebildet wird und die Schritte des Ausbildens eines tiefgeprägten Symbols und Ausbildens einer Mehrzahl abgewinkelter Oberflächen (25) im Wesentlichen gleichzeitig in einem einzigen Formungsschritt stattfinden.

10. Verfahren nach Anspruch 9, wobei die Form selektiv poliert oder aufgeraut wird, um eine andere Oberflächenrauigkeit in der Mehrzahl abgewinkelter Oberflächen (25) im Vergleich zu der glatten Oberfläche (26) zu erzeugen.

11. Verfahren nach Anspruch 8, wobei das Symbol durch maschinelle Bearbeitung ausgebildet wird und die Schritte des Ausbildens eines tiefgeprägten Symbols und Ausbildens einer Mehrzahl abgewinkelter Oberflächen (25) im Wesentlichen gleichzeitig in einem einzigen Bearbeitungsschritt stattfinden.

12. Verfahren nach Anspruch 11, ferner umfassend das selektive Polieren oder Aufrauen der Mehrzahl abgewinkelter Oberflächen (25) oder der glatten Oberfläche (26) nach dem Ausbilden des Symbols, um eine andere Oberflächenrauigkeit in der glatten Oberfläche im Vergleich zu der Mehrzahl abgewinkelter Oberflächen zu erzeugen.

## Revendications

1. Instrument chirurgical (2) comprenant un symbole en creux formé dans ou sur une surface lisse (26) entourant le symbole, dans lequel le symbole comprend une pluralité de surfaces en biais sur au moins une partie de la zone du symbole, dans lequel les surfaces en biais (25) définissent une pluralité d'arêtes (22) et rainures (24) parallèles dans le symbole, et dans lequel, dans une section transversale à travers le symbole dans un plan perpendiculaire aux arêtes et rainures, des surfaces en biais alternées s'étendent depuis un creux d'une rainure vers un pic d'une arête et depuis un pic d'une arête vers un creux d'une rainure, respectivement, et dans lequel la profondeur d'un creux d'une rainure est d'au moins 0,5 mm.

2. Instrument chirurgical (2) selon la revendication 1, dans lequel l'espacement entre des pics d'arêtes adjacentes (22) est compris entre 0,5 et 2 mm.

3. Instrument chirurgical (2) selon la revendication 2, dans lequel l'espacement entre des pics d'arêtes adjacentes (22) est d'1 mm.

4. Instrument chirurgical (2) selon l'une quelconque des revendications précédentes, dans lequel la pluralité de surfaces en biais (25) ont une rugosité de surface différente de la surface lisse (26).

5. Instrument chirurgical (2) selon la revendication 4, dans lequel la rugosité de surface peut avoir une Rₐ de moins de 1,8 µm.

6. Instrument chirurgical (2) selon l'une quelconque des revendications précédentes, dans lequel au moins trois arêtes (22) sont définies par la pluralité de surfaces en biais (25).

7. Instrument chirurgical selon l'une quelconque des revendications précédentes, dans lequel le symbole ne comprend aucun marquage imprimé.

8. Méthode de marquage d'un instrument chirurgical (2) avec un symbole formé dans ou sur une surface lisse (26), la méthode comprenant :
la formation d'un symbole dans ou sur une surface lisse de l'instrument chirurgical ; et
la formation d'une pluralité de surfaces en biais (25) sur au moins une partie de la zone du symbole, dans lequel les surfaces en biais définissent une pluralité d'arêtes (22) et de rainures (24) parallèles dans le symbole, dans lequel les surfaces en biais définissent une pluralité d'arêtes et de rainures parallèles dans le symbole, les surfaces en biais définissant une pluralité d'arêtes et de rainures parallèles dans le symbole, et dans lequel, dans une section transversale à travers le symbole dans un plan perpendiculaire aux arêtes et rainures, des surfaces en biais alternées s'étendent depuis un creux d'une rainure vers un pic d'une arête et depuis un pic d'une arête vers un creux d'une rainure, respectivement, et dans lequel la profondeur d'un creux d'une rainure est d'au moins 0,5 mm.

9. Méthode selon la revendication 8, dans laquelle le symbole est formé par moulage et les étapes de formation d'un symbole et de formation d'une pluralité de surfaces en biais (25) ont lieu sensiblement simultanément dans une seule étape de moulage.

10. Méthode selon la revendication 9, dans laquelle le moule est sélectivement poli ou rugosifié pour produire une rugosité de surface différente dans la pluralité de surfaces en biais (25) en comparaison à la surface lisse (26).

11. Méthode selon la revendication 8, dans laquelle le symbole est formé par usinage et les étapes de formation d'un symbole et de formation d'une pluralité de surfaces en biais (25) ont lieu sensiblement simultanément dans une seule étape d'usinage.

12. Méthode selon la revendication 11, comprenant en outre le polissage ou la rugosification sélective de la pluralité de surfaces en biais (25) ou de la surface lisse (26) après formation du symbole pour produire une rugosité de surface différente dans la surface lisse en comparaison à la pluralité de surfaces en biais.
